# EUROPEAN PATENT SPECIFICATION

(11) **EP 1 229 018 B1**
(45) Date of publication and mention of the grant of the patent: **09.08.2006**
(21) Application number: 01300872.7
(22) Date of filing: 31.01.2001
(51) Int. Cl.: C07C 213/10, C07C 213/00

(54) **Single step hydrogenation of nitrobenzene to p-aminophenol**
Einstufige Hydrierung von Nitrobenzol zu p-Aminophenol
Hydrogénation en une étape du nitrobenzène en p-aminophénol

(43) Date of publication of application: 07.08.2002
(73) Proprietor: COUNCIL OF SCIENTIFIC AND INDUSTRIAL RESEARCH, New Delhi 100 001 (IN)
(72) Inventor: Rode, Chandrashekhar Vasant, Maharashtra (IN); Vaidya, Manisha Jagdeeshrao, Maharashtra (IN); Chaudhari, Raghunath Vitthal, Maharashtra (IN)
(74) Representative: Manaton, Ross Timothy

(56) References cited:
- EP-A- 0 041 837
- EP-A- 0 211 545
- WO-A-93/20039
- DE-A- 2 311 038
- FR-A- 1 559 841
- FR-A- 2 618 428

## Description

### Field of the invention

The present invention relates to a single step hydrogenation process for the preparation of *p*-aminophenol. More particularly, the process relates to the preparation of *p-*aminophenol in an aqueous acid medium using mono and bimetallic nickel catalysts.

### Background of the invention

*p*-Aminophenol (PAP) is a well known and very useful industrial chemical. It is used as an intermediate in the production of pharmaceuticals such as paracetamol, in the production of dyestuffs such as sulphur dyes and in making photographic chemicals.

Conventionally, PAP is prepared by hydrolysing *p*-nitrochlorobenzene to *p-*nitrophenol. Hydrogenation of *p*-nitrophenol to PAP is then carried out using Fe/HCl catalyst. In this multi-step process, quantity of iron (catalyst precursor) required is quite large, subsequently the production of iron-iron oxide sludge is large, posing a serious effluent problem. The work-up of reaction crude is cumbersome. The quantity of iron used is very important for the faster reduction rate.

An important commercial process for the preparation of *p*-aminophenol involves the catalytic hydrogenation of nitrobenzene in acidic medium using supported platinum based catalysts. In this process phenylhydroxylamine (PHA) is first formed and this intermediate immediately rearranges in the presence of acid to PAP. The major by-product formed is aniline. In actual practice, both these steps are carried out in a single reactor. The reaction mixture consists of both aqueous as well as organic phases.

Reference may be made to US patent 3,383,416; 1969 by Benner wherein the catalyst reported was Pt/C for hydrogenation of nitrobenzene to *p*-aminophenol. Greco (US Pat. 3,953,509; 1976) reported the use of molybdenum sulphide on carbon catalyst for the hydrogenation of nitrobenzene to PAP. Dunn (US Pat. 4,264,529; 1981) has reported the use of platinum on γ-alumina for the hydrogenation of nitrobenzene to yield PAP. Low temperature hydrogenation in the presence of modified catalyst system containing sulphur compound and the rearrangement step in a separate vessel has been suggested by Caskey and Chapman (US Pat. 4,415,753; 1983). Rylander et al. (US Pat. 3,715,397; 1973) disclosed a process for preparation of PAP by catalytic hydrogenation of nitrobenzene in a sulphuric acid medium in the presence of dimethylsulfoxide, using platinum oxide catalyst.

Thus, the catalysts used for hydrogenation of nitrobenzene to *p*-aminophenol reported in the literature are Pt, Pd, Ru, and PtO₂. Among these catalysts, however, Pt is the most active for this system but it is very costly. All these being noble metal the process becomes cost intensive. It also demands to use the same catalyst for several times and also to recover the metal from deactivated catalyst in order to make the process economical.

*p*-Aminophenol is an important raw material for making paracetamol which is widely used in antipyretic and analgesic drug formulations. Conventional method for preparation of *p*-aminophenol (PAP) involves reduction of nitrophenol with Fe-HCl. This process suffers from major drawbacks such as formation of large amounts of sludge (1.2kg sludge/kg product) posing serious effluent problems and cumbersome work up of reaction crude to obtain pure PAP. Alternate process for PAP involves the catalytic hydrogenation of nitrobenzene using supported platinum catalyst in presence of aqueous acid.

WO93/20039 and EP-A-0,041,837 both relate to the purification of *p*-aminophenol. However, although they state that *p*-aminophenol may be produced by the catalytic hydrogenation of nitrobenzene, they disclose no specific catalysts for this purpose.

EP-A-0,211,545 relates to the reduction of nitro compounds to amines (e.g. nitrobenzene to aniline) in the vapour phase, without using an acidic medium. The catalysts used are oxides of nickel and cobalt.

DE-A-2,311,038 relates to the preparation of diamino-phenol by reduction of nitro-aminophenol, using Raney nickel and palladium catalysts. The reaction, which is carried out without using an acidic medium, involves the direct reduction of the nitro group of the nitro-aminophenol to the amine.

In accordance with this invention, it has now been discovered that group VIII metals like nickel alone or combinations of nickel with traces of noble metals like Pt or Pd can be used as an efficient and cheaper catalyst system for hydrogenation of nitrobenzene to PAP. Complete conversion of nitrobenzene is achieved to give PAP as the major product with aniline as a side product.

### Objects of the invention

The main object of the present invention is to provide mono and bimetallic catalysts for single step hydrogenation of nitrobenzene to *p*-aminophenol, which obviates the drawbacks as detailed above.

### Summary of the invention

The present invention enables the preparation of *p*-aminophenol in a single step by hydrogenation of nitrobenzene in presence of an aqueous acid over a mono or bimetallic Ni catalyst, terminating the reaction to obtain a reaction mixture containing the product, extracting the reaction mixture with an organic solvent, separating the aqueous layer containing PAP and neutralising with ammonia solution to separate the solid product.

More specifically, the resent invention provides a process for the single step hydrogenation of nitrobenzene to *p*-aminophenol, said process comprising the steps: (a) contacting a mixture of nitrobenzene and aqueous acid with hydrogen over a mono or bimetallic catalyst containing 5 -20% nickel at a pressure of up to 700 psi (approximately 4.8 x 10⁶ N.m⁻²) at a temperature of 80-120°C for a period of 1 to 4 hours, (b) terminating the reaction to obtain product mixture, (c) removing the reaction mixture from the source of heat, (d) separating the catalyst and resin from the reaction mixture by filtration, (e) extracting the filtrate with toluene, (f) treating the aqueous layer with ammonia solution to adjust the pH of solution to 3 to 4 to partly precipitate PAP, (g) separating the solid thus obtained by filtration, (h) extracting the filtrate with toluene, (i) treating the aqueous layer with ammonia solution to pH 7 to 8 to substantially precipitate PAP, (j) washing the total solid thus obtained after first and second extractions with distilled water and drying.

Prefecably, the organic and aqueous layers are analysed for reactants and products after step (e), using GC and HPLC.

In one embodiment of the invention, the catalyst comprises 10 % Ni on a solid support selected from the group consisting of silica, ZSM-5, clay and carbon.

In another embodiment of the invention, a bimetallic catalyst comprising Ni and traces of one of the group VIII metals is used as a catalyst system to increase the selectivity of *p*-aminophenol.

In still another embodiment of the present invention, the concentration of acid used ranges between 2.5-10% w/w.

In another embodiment of the present invention, Pt content of the catalyst is in the range of 0.05-3%.

In another embodiment the organic solvent used for the extraction of reaction mixture may be selected from toluene, cyclohexane, ethyl acetate or the like.

### Detailed description of the invention

The following examples are given by way of illustration and therefore should not be construed to limit the scope of the present invention.

### EXAMPLE 1

### Preparation of powdered 10% Ni/ZSM-5 catalyst

Monometallic Ni supported on zeolite catalyst was prepared by precipitation technique following the procedure as given: The support (5 gm) zeolite ZSM-5 (Si/Al=20) was calcined for 4 hours before use at 773 K. A slurry of the support was made in distilled water, stirred for 2 hrs at 363 K. To this hot solution, 2.47 gm of Ni(NO₃)₂.6H₂O was added. After stirring for 6 hrs, 10 % ammonium carbonate solution was added dropwise. The addition continued till a pH value of 10 was attained. The resulting slurry was filtered to obtain a green cake and a colourless filtrate confirming the complete precipitation of Ni as nickel carbonate. The AAS analysis revealed the absence of Ni in the filtrate. The cake was dried overnight at 383 K and was calcined in a static air furnace at 773 K for 10 hrs. The reduction was carried out in an activation furnace using Silica-quartz tube at 773 K at hydrogen flow rate of 5x10⁻⁵ m³/min. for 10 hrs.

### EXAMPLE 2

For bimetallic catalyst, the monometallic 10% Ni/ZSM-5 catalyst before calcination is charged to a toluene solution containing Pt (C₂H₁₂)Cl₂ as Pt precursor. This suspension was refluxed for 4 hrs and the excess toluene was removed using a rotavapor. The powder obtained was calcined in static air at 773 K for 10 hrs. The reduction of the catalyst was carried out in an activation furnace using Silica-quartz tube at 773 K at hydrogen flow rate of 5x10⁻⁵ m³/min for 10 hrs.

### EXAMPLE 3

PAP was prepared by single step catalytic hydrogenation of nitrobenzene in a stirred 0.3 litre hastelloy autoclave having an automatic temperature contraller. A reaction charge was prepared by adding 10 gm (0.0813 mol) of nitrobenzene, 85gm water, 0.20 gm 10%Ni/C catalyst and 5.5 gm (3ml) sulphuric acid. The reactor was sealed, purged initially with nitrogen and then with hydrogen. When the reaction temperature was attained to 120°C, the reactor was pressurised to 400 psig (approximately 2.7x10⁶ N.m⁻² gauge) with H₂ and the reaction was commenced by starting the agitation. The temperature was controlled during the reaction in the range of 118-122°C. Hydrogen uptake was monitored with pressure gauge as a function of time. When hydrogen uptake stopped abruptly indicating the end of reaction (after slightly more than 2 moles of hydrogen per mole of nitrobenzene was consumed), the reaction was stopped. After completion of reaction, the reactor was purged with nitrogen, the reaction mixture was removed from the autoclave and the catalyst was separated from the reaction mixture by filtration. The fibrate was extracted with toluene. The organic and aqueous layers were analysed for reactants and products using GC and HPLC. The aqueous layer was treated with ammonia solution to adjust the pH of solution to 3-4, when PAP is precipitated partly. The solid thus obtained is separated by filtration. Again the filtrate is extracted with toluene and aqueous layer is treated with ammonia solution to pH 7-8 when maximum amount of PAP is precipitated. The total solid thus obtained after first and second extraction is washed with distilled water, dried and weighed. The conversion of nitrobenzene was found to be 14% and selectivity of PAP was found to be 14% and 86% to aniline.

### EXAMPLE 4

In a typical experiment, 10 gm of nitrobenzene, 85 gm of water, 0.2 gm of 10 %Ni/SiO₂ catalyst and 3 ml sulphuric acid were added to the reactor. The reaction was carried out at 120°C and hydrogen pressure of 400 psig (approximately 2.7x10⁶ N.m⁻² gauge). After completion of reaction the reactor was cooled, catalyst was separated by filtration and washed and extracted with toluene. The solid PAP was recovered from aqueous layer by neutralisation with ammonia solution. Aqueous and organic layers were analysed by GC and HFLC. The conversion of nintrobenzene was found to be 30% with 11% selectivity to PAP and 89% selectivity to aniline.

### EXAMPLE 5

In a typical experiment, 10 gm of nitrobenzene, 85 gm of water, 0.2 gm of 10 %Ni/ZSM-5 catalyst and 3 ml sulphuric acid were added to the reactor. The reaction was carried out at 120°C and hydrogen pressure of 400 psig (approximately 2-7x10⁶ N.m⁻² gauge). After completion of reaction the reactor was cooled, catalyst was separated by filtration, washed and extracted with toluene. The solid PAP was recovered from aqueous layer by neutralisation with ammonia solution. Aqueous and organic layers were analysed by GC and HPLC. The conversion of nitrobenzene was found to be 45 % with 45 % selectivity to PAP and 55% selectivity to aniline.

### EXAMPLE 6

In a typical experiment, 10 gm of nitrobenzene, 85 gm of water, 0.2 gm of 10 %Ni-0.05 %Pt/ZSM-5 catalyst and 3 ml sulphuric acid were added to the reactor. The reaction was caried out at 120°C and hydrogen pressure of 400 psig (approximately 2.7x10⁶ N.m⁻² gauge). After completion of reaction the reactor was cooled, catalyst was separated by filtration and washed and extracted with toluene. The solid PAP was recovered from aqueous layer by neutralisation with ammonia solution. Aqueous and organic layers were analysed by GC and HPLC. The conversion of nitrobenzene was found to be 49% with 11% selectivity to PAP and rest to aniline.

### EXAMPLE 7

In a typical experiment, 10 gm of nitrobenzene, 85 gm of water, 0.2 gm of 10 %Ni-0.1%Pt/ZSM-5 catalyst and 3 ml sulphuric acid were added to the reactor. The reaction was carried out at 120°C and hydrogen pressure of 400 psig (approximately 2.7x10⁶ N.m⁻² gauge). After completion of reaction the reactor was cooled, catalyst was separated by filtration and washed and extracted with toluene The solid PAP was recovered from aqueous layer by neutralisation with ammonia solution. Aqueous and organic layers were analysed by GC and HPLC. The conversion of nitrobenzene was found to be 42 % with 25% selectivity to PAP and 75 % selectivity to aniline.

### EXAMPLE 8

In a typical experiment, 10 gm of nitrobenzene, 85 gm of water, 0.2 gm of 10 %Ni-1%Pt/ZSM-5 catalyst and 3 ml sulphuric acid were added to the reactor. The reaction was carried out at 120°C and hydrogen pressure of 400 psig (approximately 2.7x10⁶ N.m⁻² gauge). After completion of reaction the reactor was cooled, catalyst was separated by filtration and washed and extracted with toluene. The solid PAP was recovered from aqueous layer by neutralisation with ammonia solution Aqueous and organic layers were analysed by GC and HPLC. The conversion of nitrobenzene was found to be 93% with 63 % selectivity to PAP and 45% selectivity to aniline.

### EXAMPLE 9

In a typical experiment, 10 gm of nitrobenzene, 85 gm of water, 02 gm of 10 %Ni-1%Pt/ZSM-5 catalyst and 3 ml sulphuric acid were added to the reactor. The reaction was carried out at 80°C and hydrogen pressure of 400 psig (approximately 2.7x10⁶ N.m⁻² gauge). After completion of reaction the reactor was cooled, catalyst was separated by filtration and washed and extracted with toluene. The solid PAP was recovered from aqueous layer by neutralisation with ammonia solution. Aqueous and organic layers were analysed by GC and HPLC. The conversion of nitrobenzene was found to be 100% with 25 % selectivity to PAP and 75 % selectivity to aniline.

### EXAMPLE 10

In a typical experiment, 10 gm of nitrobenzene, 85 gm of water, 0.1 gm of 10 %Ni-1%Pt/ZSM-5 catalyst and 3 ml sulphuric acid were added to the reactor. The reaction was carried out at 120°C and hydrogen pressure of 400 psig (approximately 2.7x10⁶ N.m⁻² gauge). After completion of reaction the reactor was cooled, catalyst was separated by filtration and washed and extracted with toluene. The solid PAP was recovered from aqueous layer by neutralisation with ammonia solution. Aqueous and organic layers were analysed by GC and HPLC. The conversion of nitrobenzene was found to be 99 % with 45% selectivity to PAP and 55% selectivity to aniline.

### EXAMPLE 11

In a typical experiment, 10 gm of nitrobenzene, 85 gm of water, 0.1 gm of 10%Ni-3 %Pt/ZSM-5 catalyst and 3 ml sulphuric acid were added to the reactor. The reaction was carried out at 120°C and hydrogen pressure of 400 psig (approximately 2.7x10⁶ N.m⁻² gauge). After completion of reaction the reactor was cooled, catalyst was separated by filtration and washed and extracted with toluene. The solid PAP was recovered from aqueous layer by neutralisation with ammonia solution. Aqueous and organic layers were analysed by GC and HPLC. The conversion of nitrobenzene was found to be 99 % with 65 % selective to PAP and 35 % selectivity to aniline

### EXAMPLE 12

In a typical experiment, 10 gm of nitrobenzene, 85 gm of water, 0.1 gm of 10 %Ni -1 %Pd/ZSM-5 catalyst and 3 ml sulphuric acid were added to the reactor. The reaction was carried out at 120°C and hydrogen pressure of 400 psig (approximately 2.7x10⁶ N.m⁻² gauge). After completion of reaction the completion of reaction the reactor was cooled, catalyst was separated by filtration and washed and extracted with toluene. The solid PAP was recovered from aqueous layer by neutralisation with ammonia solution. Aqueous and organic layers were analysed by GC and HPLC. The conversion of nitrobenzene was found to be 99 % with 20 % selectivity to PAP and 79 % selectivity to aniline.

## Claims

1. A process for the single step hydrogenation of nitrobenzene to *p*-aminophenol, said process comprising the steps:
(a) contacting a mixture of nitrobenzene and aqueous acid with hydrogen over a mono or bimetallic catalyst containing 5-20% nickel, at a pressure of up to 700 psi (approximately 4.8 x 10⁶N.m⁻²) at a temperature of 80-120°C for a period of 1 to 4 hours,
(b) terminating the reaction to obtain product mixture,
(c) removing the reaction mixture from the source of heat,
(d) separating the catalyst and resin from the reaction mixture by filtration,
(e) extracting the filtrate with toluene,
(f) treating the aqueous layer with ammonia solution to adjust the pH of solution to 3 to 4 to partly precipitate PAP,
(g) separating the solid thus obtained by filtration,
(h) extracting the filtrate with toluene,
(i) treating the aqueous layer with ammonia solution to pH 7 to 8 to substantially precipitate PAP,
(j) washing the total solid thus obtained after first and second extractions with distilled water and drying.

2. A process as claimed in claim 1, wherein the temperature of the reaction ranges between 80-120°C.

3. A process as claimed in claim 1, wherein the catalyst further contains Platinum or Palladium.

4. A process as claimed in claim 3, wherein the Pt content of the catalyst is in the range of 0.05-3%.

## Patentansprüche

1. Verfahren zur einstufigen Hydrierung von Nitrobenzol zu p-Aminophenol, wobei das Verfahren die folgenden Stufen umfasst:
a) Kontaktierung eines Gemisches aus Nitrobenzol und einer wässrigen Säure mit Wasserstoff über einem 5-20 % Nickel enthaltenden mono- oder bimetallischen Katalysator bei einem Druck von bis zu 700 psi (ca. 4,8 x 10⁶N.m⁻²) bei einer Temperatur von 80-120°C während 1 bis 4 Stunden,
b) Beendigung der Reaktion zur Erzielung eines Produktgemisches,
c) Entfernung des Reaktionsgemisches aus der Wärmequelle,
d) Abtrennung des Katalysators und des Harzes aus dem Reaktionsgemisch durch Filtration,
e) Extraktion des Filtrats mit Toluol,
f) Behandlung der wässrigen Schicht mit einer Ammoniaklösung zur Einstellung des pH der Lösung auf 3 bis 4 bis zur teilweisen Ausfällung von PAP,
g) Abtrennung des durch Filtration erhaltenen Feststoffs,
h) Extraktion des Filtrats mit Toluol,
i) Behandlung der wässrigen Schicht mit einer Ammoniaklösung bis zu einem pH von 7 bis 8 bis zur praktisch vollständigen Ausfällung des PAP und
j) Waschen des nach der ersten und zweiten Extraktion erhaltenen Gesamtfeststoffs mit destilliertem Wasser und Trocknung.

2. Verfahren nach Anspruch 1, bei dem die Reaktionstemperatur in einem Bereich zwischen 80 und 120°C liegt.

3. Verfahren nach Anspruch 1, bei dem der Katalysator außerdem noch Platin oder Palladium enthält.

4. Verfahren nach Anspruch 3, bei dem der Pt-Gehalt des Katalysators in einem Bereich von 0.05-3 % liegt.

## Revendications

1. Procédé d'hydrogénation en une étape de nitrobenzène en p-aminophénol, ledit procédé comprenant les étapes consistant à :
(a) mettre en contact un mélange de nitrobenzène et d'acide aqueux avec de l'hydrogène sur un catalyseur mono- ou bimétallique contenant 5 à 20 % de nickel, à une pression allant jusqu'à 700 psi (approximativement 4,8 x 10⁶ N·m⁻²) à une température de 80 à 120 °C pendant une durée de 1 à 4 heures,
(b) terminer la réaction pour obtenir le mélange des produits,
(c) retirer le mélange réactionnel de la source de chaleur,
(d) séparer le catalyseur et la résine du mélange réactionnel par filtration,
(e) extraire le filtrat avec du toluène,
(f) traiter la phase aqueuse avec une solution d'ammoniac pour ajuster le pH de la solution entre 3 et 4 pour précipiter partiellement le PAP,
(g) séparer les solides ainsi obtenus par filtration,
(h) extraire le filtrat avec du toluène,
(i) traiter la phase aqueuse avec une solution d'ammoniac à pH 7 à 8 pour précipiter le PAP pratiquement complètement,
(j) laver la totalité des solides ainsi obtenus après une première et une seconde extractions avec de l'eau distillée et sécher.

2. Procédé selon la revendication 1, dans lequel la température de la réaction est dans la gamme entre 80 et 120 °C.

3. Procédé la revendication 1, dans lequel le catalyseur contient en outre du platine ou du palladium.

4. Procédé la revendication 3, dans lequel la teneur en Pt du catalyseur est dans la gamme de 0,05 à 3 %.
